# FASCICULE DE BREVET EUROPEEN

(11) **EP 0 759 031 B1**
(45) Date de publication et mention de la délivrance du brevet: **12.08.1998**
(21) Numéro de dépôt: 95919484.6
(22) Date de dépôt: 05.05.1995
(51) Int. Cl.: C07K 5/072

(54) **PROCEDE PERFECTIONNE DE PREPARATION D'UN COMPOSE DERIVE DE L'ASPARTAME UTILE COMME AGENT EDULCORANT**
VERBESSERTES VERFAHREN ZUR HERSTELLUNG AUS EINER VON ASPARTAN ABSTAMMENDEN VERBINDUNGEN, ALS SÜSSSTOFF VERWENDBAR
IMPROVED METHOD FOR THE PREPARATION OF AN ASPARTAME-DERIVED COMPOUND USEFUL AS A SWEETENER

(30) Priorité: 09.05.1994 FR 9405674
(43) Date de publication de la demande: 26.02.1997
(73) Titulaire: NOFRE, Claude, F-69003 Lyon (FR); TINTI, Jean-Marie, F-69680 Chassieu (FR)
(72) Inventeur: NOFRE, Claude, F-69003 Lyon (FR); TINTI, Jean-Marie, F-69680 Chassieu (FR)
(74) Mandataire: Hubert, Philippe
(86) Numéro de dépôt international: FR9500589
(87) Numéro de publication internationale: WO9530689

(56) Documents cités:
- WO-A-94/11391

## Description

La présente invention a pour objet un procédé perfectionné de préparation d'un composé dérivé de l'aspartame utile comme agent édulcorant.

Plus précisément, la présente invention a pour objet un procédé de préparation d'un dérivé *N*-substitué de l'aspartame, à savoir le *N*-[*N*-(3,3-diméthylbutyl)-L-α-aspartyl]-L-phénylalanine 1-méthyl ester de formule :

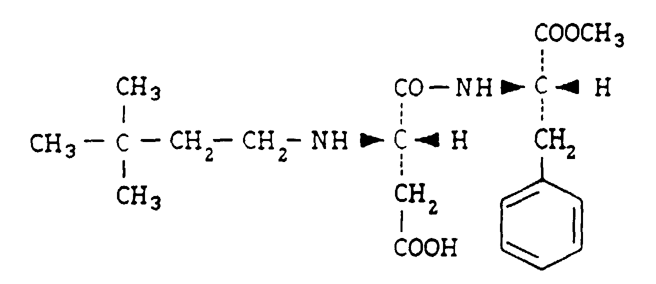

Ce composé constitue un édulcorant très puissant puisqu'il présente sur une base pondérale un pouvoir sucrant qui est au moins 50 (cinquante) fois plus élevé que celui de l'aspartame et environ 10 000 (dix mille) fois plus élevé que celui du saccharose (sucre de table).

Les édulcorants étant principalement destinés à la consommation alimentaire humaine, il est nécessaire de disposer, pour leur préparation, de procédés permettant l'obtention de produits de grande pureté pratiquement exempts de contaminants ou de produits de dégradation.

En outre, pour être utilisables à l'échelle industrielle, ces procédés doivent être parfaitement maîtrisés pour être reproductibles, et d'un coût relativement faible.

Dans la mesure où le composé dont le procédé de préparation fait l'objet de la présente invention est un dérivé de l'aspartame, il apparaît avantageux de rechercher une voie de synthèse utilisant l'aspartame comme composé de départ ou comme composé intermédiaire.

L'aspartame, en effet, étant actuellement l'édulcorant de synthèse le plus largement utilisé, il répond aux normes requises pour une utilisation alimentaire; de plus, sa préparation industrielle est parfaitement maîtrisée avec un coût relativement bas en dépit de sa structure dipeptidique.

Cependant, on sait qu'il est relativement difficile d'utiliser l'aspartame comme produit de départ ou comme produit intermédiaire dans une synthèse industrielle.

L'aspartame, en effet, présente une solubilité relativement faible dans la plupart des solvants organiques, généralement inférieure à quelques grammes par litre.

Par ailleurs, si la solubilité de l'aspartame est plus élevée dans les milieux aqueux, sa stabilité est cependant relativement faible dans ces milieux.

En outre, toute tentative d'élévation de la température dans le but d'améliorer la solubilité de l'aspartame aggrave ses processus de dégradation.

Dans ces conditions, la présente invention a pour but de résoudre le problème technique consistant en la fourniture d'un procédé perfectionné de préparation du dérivé précité de l'aspartame qui puisse être mis en oeuvre de façon aisée et reproductible à l'échelle industrielle à un prix de revient relativement bas, et qui utilise l'aspartame comme produit de départ ou comme produit intermédiaire.

Il a été découvert, et ceci constitue le fondement de la présente invention, qu'il était possible d'obtenir le dérivé *N*-alkylé de l'aspartame précité directement à partir de l'aspartame, en une seule étape, et ce avec un rendement extrêmement élevé et une très grande pureté compatible avec l'utilisation de ce composé dans le domaine alimentaire.

Selon la présente invention, le procédé de préparation du *N*-[*N*-(3,3-diméthylbutyl)-L-α-aspartyl]-L-phénylalanine 1-méthyl ester de formule : 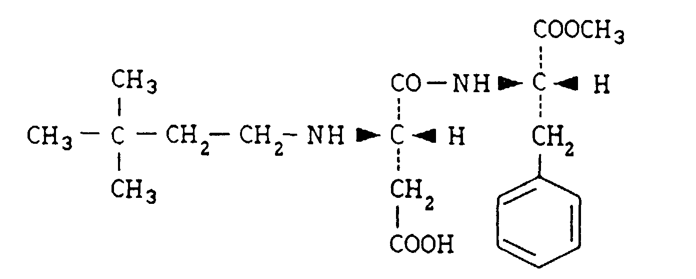 est caractérisé en ce que l'on traite une solution d'aspartame et de 3,3-diméthylbutyraldéhyde à température ambiante par de l'hydrogène à une pression inférieure ou égale à 1 bar (0,1 MPa) en présence d'un catalyseur à base de platine ou de palladium.

Dans une forme avantageuse de réalisation de l'invention, le catalyseur précité est choisi dans le groupe comprenant le platine sur carbone activé, le palladium sur carbone activé, le noir de platine et le noir de palladium.

Dans une forme particulièrement avantageuse, l'hydrogénation est réalisée en présence de platine à 5 % sur carbone activé à la pression relative de 1 bar, ou en présence de palladium à 10 % sur carbone activé à la pression atmosphérique ou à la pression relative de 1 bar.

Dans une mise en oeuvre actuellement préférée de l'invention, la solution d'aspartame et de 3,3-diméthylbutyraldéhyde est une solution hydroalcoolique de pH 4,5-5 obtenue par mélange d'une solution d'acide acétique 0,1 M et de méthanol, la concentration d'aspartame étant dans cette solution comprise entre 50 et 60 g/L et celle de 3,3-diméthylbutyraldéhyde entre 20 et 30 g/L.

Dans un aspect avantageux de l'invention, le produit formé est purifié par précipitation et filtration après élimination sous vide de la partie alcoolique du solvant.

Le procédé conforme à l'invention permet ainsi d'obtenir, par N-alkylation réductive de l'aspartame, un composé édulcorant très pur, avec un rendement très élevé.

Il est décrit dans la littérature de nombreux exemples de N-alkylation réductive qui mettent en oeuvre l'hydrogène associé à des catalyseurs (voir par exemple la revue de P. N. Rylander, "Catalytic Hydrogenation in Organic Syntheses", Academic Press, San Diego, 1993, pp. 165-174). L'application de cette technique générale au procédé décrit dans la présente invention n'a cependant pu être réalisée qu'en sélectionnant les catalyseurs et en adoptant des conditions expérimentales très spécifiques qui ont seules conduit à la haute pureté analytique nécessaire pour l'utilisation alimentaire du composé faisant l'objet du procédé de la présente invention.

Il a été en effet constaté que la qualité du produit désiré dépendait très étroitement des conditions expérimentales appliquées durant la mise en oeuvre du procédé. La nature du catalyseur, et dans une moindre mesure la durée et la pression d'hydrogénation, la nature du milieu réactionnel et son pH se sont ainsi révélés être des paramètres essentiels.

Il est à noter que dans le cas des réactions d'hydrogénation à l'échelle industrielle, les conditions qui permettent d'abaisser notablement le temps de réaction jusqu'à des durées de l'ordre de quelques heures, tout en conservant un rendement satisfaisant et en restant dans des domaines de pressions inférieures ou égales à 1 bar (0,1 MPa), sont très recherchées. Bien qu'accélérant généralement la réaction, l'utilisation de hautes pressions n'est généralement pas souhaitée pour des raisons de sécurité et de coût du matériel.

Les catalyseurs d'hydrogénation utilisés dans le cadre de la présente invention agissent d'une façon inattendue d'une part à des pressions relatives inférieures ou égales à 1 bar (0,1 MPa) et d'autre part dans des durées inférieures ou égales à 24 heures.

Un suivi de l'état d'avancement de la réaction par prélèvement et évaluation du produit formé par chromatographie liquide haute performance (H.P.L.C.) permet à l'homme de l'art de déterminer aisément la durée d'hydrogénation la plus appropriée aux conditions utilisées.

Parmi les catalyseurs possibles, ceux à base de platine ou de palladium dispersés sur du carbone activé ou sous forme de noir de platine ou de palladium se sont révélés particulièrement avantageux.

Il a été observé que d'autres catalyseurs comme le nickel sur silice (Aldrich n° 20,878-7), le nickel sur silice et alumine (Aldrich n° 20,877-9), le nickel Raney (Aldrich n° 22,167-8), le noir de ruthénium (Aldrich n° 32,671-2), le ruthénium sur carbone (Aldrich n° 28,147-6), l'hydroxyde de palladium sur carbone (Aldrich n° 21,291-1), l'oxyde de palladium (Aldrich n° 20,397-1), le noir de rhodium (Aldrich n° 26,734-1), le rhodium sur carbone (Aldrich n° 33,017-5) ou le rhodium sur alumine (Fluka n° 83720) permettent également la préparation du composé de l'invention. Ces catalyseurs se sont cependant révélés soit moins actifs en nécessitant notamment des pressions d'hydrogène plus élevées, soit moins sélectifs en conduisant notamment à une réduction du cycle aromatique porté par l'aspartame ou par son dérivé N-alkylé recherché.

Il a été également observé que des pressions d'hydrogénation plus élevées ou des durées d'hydrogénation plus longues que celles utilisées dans le procédé de l'invention peuvent aussi affecter le rendement et la qualité du produit fini. Il en est de même pour les quantités de catalyseur utilisées qui influent aussi notablement sur les temps d'hydrogénation.

Les catalyseurs sélectionnés dans le cadre de la présente invention se sont révélés particulièrement efficaces à des concentrations comprises entre 5 et 20 % par rapport à l'aspartame.

La solution hydroalcoolique de pH 4,5-5 utilisée dans le procédé de l'invention s'est révélée particulièrement avantageuse en permettant une dissolution rapide des réactifs et en favorisant au cours du traitement la séparation du produit désiré dans un état de grande pureté. L'utilisation d'un milieu réactionnel uniquement aqueux provoque en effet la précipitation du produit et son agrégation avec le catalyseur. Les durées de réaction sont alors plus longues et la séparation du catalyseur difficile.

Il a été de plus constaté qu'un pH voisin de 4,5-5 pour le milieu réactionnel accélérait la réaction tout en diminuant notablement les processus de dégradation de l'aspartame.

En résumé, le choix de catalyseurs très spécifiques opérant sous de faibles pressions d'hydrogène, dans un temps souvent très court, à la température ambiante et en milieu hydroalcoolique de pH 4,5-5, permet de respecter les contraintes de stabilité et de solubilité liées à l'aspartame. Les rendements bruts élevés obtenus dans ces conditions facilitent l'obtention d'un produit de très grande qualité qui, de plus, est aisément récupéré par simple précipitation après élimination de la partie alcoolique du solvant réactionnel.

La présente invention sera plus complètement décrite à l'aide des exemples suivants qui ne doivent pas être considérés comme limitatifs de l'invention.

### EXEMPLE 1

Dans un réacteur équipé d'une agitation permettant d'assurer un très bon transfert de l'hydrogène gazeux dans la phase liquide, on introduit sous agitation et dans l'ordre : 60 cm³ d'une solution aqueuse d'acide acétique 0,1 M, 1 g de platine à 5 % sur carbone activé (produit Aldrich n° 33,015-9 : Platinum on activated carbon, wet, Degussa type F101 RA/W, Pt 5%), 2,55 g de 3,3-diméthylbutyraldéhyde, 30 cm³ de méthanol et 5 g d'aspartame.

Après avoir purgé le réacteur par un courant d'azote, le mélange est soumis à une hydrogénation à la pression relative de 1 bar (0,1 MPa) et à température ambiante. L'avancement de la réaction est contrôlé par prélèvement d'un échantillon brut et dosage du produit formé par chromatographie liquide haute performance (H.P.L.C.). La concentration en produit désiré est déterminée par comparaison avec une courbe d'étalonnage préalablement établie. Après 2 heures d'hydrogénation, on observe la formation de 100 % du produit attendu.

La réaction est alors interrompue en purgeant le réacteur par un courant d'azote et en séparant le catalyseur par filtration sur filtre fin (0,5 µm). Si besoin est, le filtrat est ajusté à pH 5 par addition de quelques gouttes d'une solution d'hydroxyde de sodium 1 N. Le méthanol est alors éliminé par évaporation sous vide, la température étant maintenue au-dessous de 40 °C. Un solide blanc précipite rapidement. Le mélange est encore agité quelques heures à température ambiante pour compléter la précipitation. Le produit est séparé par filtration, séché et lavé par environ 50 cm³ d'hexane. On obtient finalement 4,4 g de *N*-[*N-*(3,3-diméthylbutyl)-L-α-aspartyl] -L-phénylalanine 1-méthyl ester (rendement 69 %) sous la forme d'une poudre blanche de grande pureté (supérieure à 98 % par H.P.L.C.).

### EXEMPLE 2

En utilisant le même appareillage, le même solvant et les mêmes réactifs aux mêmes concentrations que ceux décrits dans l'exemple 1, mais en employant comme catalyseur 1 g de palladium à 10 % sur carbone activé (produit Fluka n° 75990 ; Palladium on activated charcoal; 10 % Pd), en effectuant l'hydrogénation à une pression relative de 1 bar (0,1 MPa), toujours à température ambiante, la réaction est arrêtée après 2 heures (96 % de produit formé). Après purification par précipitation suivant le protocole décrit dans l'exemple 1, on obtient 4,3 g du produit attendu (rendement 68 %) sous forme de poudre blanche de très grande pureté (supérieure à 98 % par H.P.L.C.).

### EXEMPLE 3

En utilisant le même appareillage, le même solvant et les mêmes réactifs aux mêmes concentrations que ceux décrits dans l'exemple 1, mais en employant comme catalyseur 1 g de palladium à 10 % sur carbone activé (produit Fluka n° 75990 : Palladium on activated charcoal; 10 % Pd), en effectuant l'hydrogénation à la pression atmosphérique, toujours à température ambiante, la réaction est arrêtée après 24 heures (97 % de produit formé). Après purification par précipitation suivant le protocole décrit dans l'exemple 1, on obtient 4,3 g du produit attendu (rendement 68 %) sous forme de poudre blanche de très grande pureté (supérieure à 98 % par H.P.L.C.).

### EXEMPLE 4

En utilisant le même appareillage, le même solvant et les mêmes réactifs aux mêmes concentrations que ceux décrits dans l'exemple 1, mais en employant comme catalyseur 1 g de noir de platine (produit Aldrich n° 20,591-5 : Platinum black) , en effectuant l'hydrogénation à la pression atmosphérique, toujours à température ambiante, la réaction est arrêtée après 1 heure (96 % de produit formé). Après purification par précipitation suivant le protocole décrit dans l'exemple 1, on obtient 4,4 g du produit attendu (rendement 69 %) sous forme de poudre blanche de très grande pureté (supérieure à 98 % par H.P.L.C.).

### EXEMPLE 5

En utilisant le même appareillage, le même solvant et les mêmes réactifs aux mêmes concentrations que ceux décrits dans l'exemple 1, mais en employant comme catalyseur 1 g de noir de palladium (produit Aldrich n° 20,583-4 : Palladium black), en effectuant l'hydrogénation à la pression atmosphérique, toujours à température ambiante, la réaction est arrêtée après 16 heures (98 % de produit formé). Après purification par précipitation suivant le protocole décrit dans l'exemple 1, on obtient 4,4 g du produit attendu (rendement 69 %) sous forme de poudre blanche de très grande pureté (supérieure à 98 % par H.P.L.C.).

La pureté du composé préparé suivant le procédé de l'invention est contrôlée par les techniques classiques de chromatographie sur couche mince, spectrométrie en infrarouge, spectrométrie en ultraviolet, chromatographie liquide haute performance (H.P.L.C.), analyse thermique, pouvoir rotatoire, résonance magnétique nucléaire et analyse centésimale.

Les critères physiques obtenus pour le composé préparé selon l'invention sont donnés ci-après.

Poudre blanche amorphe, inodore, non hygroscopique.

Formule moléculaire : C₂₀H₃₀N₂O_{5.}

Poids moléculaire : 378,4.

Teneur en eau (méthode de Karl Fischer) : 3 à 6 %.

Chromatographie sur couche mince : Gel de silice 60 F254 sur feuilles d'aluminium (Merck n° 5554), éluant : butanol-acide acétique-eau (8:2:2), révélation à la ninhydrine : Rf : 0,54.

Spectre infrarouge (KBr) cm⁻¹ : 3587 (HOH), 3444, 3319 (NH), 3028 (CH), 2957, 2867 (CH), 1733 (COOCH₃), 1690 (CONH), 1594 (COO⁻), 1565, 1541, 1440, 1414, 1390, 1368, 1278, 1245, 1218, 1173, 1119, 999, 758, 701 (CH).

Spectre ultraviolet : maximums à 214 nm et 257 nm.

Chromatographie liquide haute performance sur colonne Merck de type "Lichrospher 100 RP-18 endcapped", longueur 244 mm, diamètre 4 mm, éluant : acétate d'ammonium 65 mM - acétonitrile (65:35), débit : 1 ml/min, détecteur : réfractomètre, temps de rétention 7,7 min.

Analyse thermique différentielle de 40 à 350 °C à 10 °C/min : point de fusion 84 °C, pas de décomposition au-dessous de 200 °C.

Pouvoir rotatoire : [α]^{D}₂₀ = 46,5 ° ± 1,5 (c = 2, méthanol).

Spectre de résonance magnétique nucléaire (H, 200 MHz, DMSO-d6) 0,81 (s, 9 H), 1,28 (m, 2 H), 2,38 (m, 4 H), 2,9 (m, 2 H), 3,44 (m, 1 H), 3,62 (s, 3 H), 4,55 (m, 1 H), 7,22 (m, 5 H), 8,54 (d, 1 H).

Analyse centésimale : trouvé (théorique pour 4,5 % d'eau) :

C 60,51 (60,73) H 7,86 (8,12) N 7,07 (7,08) O 23,62 (24,04)

Le composé préparé suivant le procédé qui vient d'être décrit et illustré est particulièrement utile pour édulcorer des produits variés, en particulier les boissons, les aliments, les confiseries, les pâtisseries, les chewing-gums, les produits d'hygiène et les articles de toilette, ainsi que les produits cosmétiques, pharmaceutiques et vétérinaires.

## Revendications

1. Procédé de préparation du *N*-[*N*-(3,3-diméthylbutyl)-L-α-aspartyl]-L-phénylalanine 1-méthyl ester de formule : 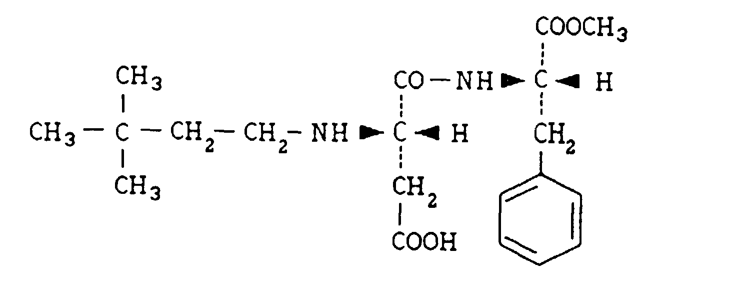 caractérisé en ce que l'on traite une solution d'aspartame et de 3,3-diméthylbutyraldéhyde à température ambiante par de l'hydrogène à une pression relative égale ou inférieure à 1 bar (0,1 MPa) en présence d'un catalyseur à base de platine ou de palladium.

2. Procédé selon la revendication 1 caractérisé en ce que le catalyseur est choisi dans le groupe comprenant le platine sur carbone activé, le palladium sur carbone activé, le noir de platine et le noir de palladium.

3. Procédé selon la revendication 1 caractérisé en ce que l'hydrogénation est réalisée en présence de platine à 5 % sur carbone activé à la pression relative de 1 bar.

4. Procédé selon la revendication 1 caractérisé en ce que l'hydrogénation est réalisée en présence de palladium à 10 % sur carbone activé à la pression relative de 1 bar ou à la pression atmosphérique.

5. Procédé selon la renvendication 1 caractérisé en ce que l'hydrogénation est réalisée en présence de noir de platine ou de noir de palladium à la pression atmosphérique.

6. Procédé selon la revendication 1 caractérisé en ce que la solution d'aspartame et de 3,3-diméthylbutyraldéhyde est une solution hydroalcoolique de pH 4,5-5 obtenue par mélange d'une solution d'acide acétique 0,1 M et de méthanol.

7. Procédé selon les revendications 1 à 6 caractérisé en ce que la concentration d'aspartame dans le solvant hydroalcoolique est comprise entre 50 et 60 g/L et la concentration de 3,3-diméthylbutyraldéhyde est comprise entre 20 et 30 g/L.

8. Procédé selon les revendications 1 à 7 caractérisé en ce que le produit formé est purifié par précipitation et filtration après élimination sous vide de la partie alcoolique du solvant.

## Patentansprüche

1. Verfahren zur Herstellung von N-[N-(3,3-Dimethylbutyl)-L-a-aspartyl]-L-phenylalanin-l-methylester der Formel: 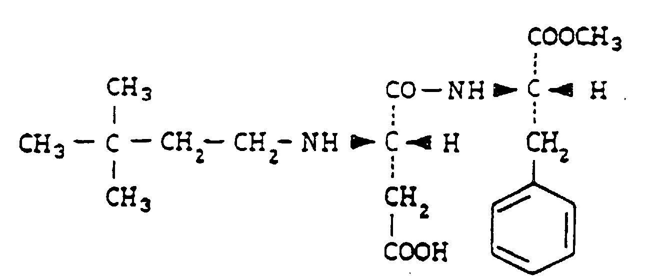 dadurch gekennzeichnet, daß eine Lösung von Aspartam und 3,3-Dimethylbutyraldehyd bei Umgebungstemperatur mit Wasserstoff bei einem relativen Druck von kleiner oder gleich 1 bar (0,1 MPa) in Anwesenheit eines Katalysators auf der Basis von Platin oder Palladium behandelt wird.

2. Verfahren nach Anspruch 1, dadurch gekennzeichnet, daß der Katalysator ausgewählt wird aus der Gruppe umfassend Platin-auf-Aktivkohle, Palladium-auf-Aktivkohle, Platinschwarz und Palladiumschwarz.

3. Verfahren nach Anspruch 1, dadurch gekennzeichnet, daß die Hydrierung in Anwesenheit von 5 % Platin-auf-Aktivkohle bei einem relativen Druck von 1 bar durchgeführt wird.

4. Verfahren nach Anspruch 1, dadurch gekennzeichnet, daß die Hydrierung in Anwesenheit von 10 % Palladium-auf-Aktivkohle bei einem relativen Druck von 1 bar oder bei Atmosphärendruck durchgeführt wird.

5. Verfahren nach Anspruch 1, dadurch gekennzeichnet, daß die Hydrierung in Anwesenheit von Platinschwarz oder Palladiumschwarz bei Atmosphärendruck durchgeführt wird.

6. Verfahren nach Anspruch 1, dadurch gekennzeichnet, daß die Lösung von Aspartam und 3,3-Dimethylbutyraldehyd eine hydroalkoholische Lösung mit einem pH von 4,5 bis 5 ist, die durch Mischen einer Lösung von 0,1 M Essigsäure und Methanol erhalten wird.

7. Verfahren nach einem der Ansprüche 1 bis 6, dadurch gekennzeichnet, daß die Aspartam-Konzentration im hydroalkoholischen Lösungsmittel zwischen 50 und 60 g/l beträgt, und die Konzentration von 3,3-Dimethylbutyraldehyd zwischen 20 und 30 g/l beträgt.

8. Verfahren nach einem der Ansprüche 1 bis 7, dadurch gekennzeichnet, daß das gebildete Produkt durch Ausfällung und Filtration gereinigt wird, nachdem der Alkohol-Teil des Lösungsmittels im Vakuum eliminiert wurde.

## Claims

1. A method of preparing *N*-[*N*-(3,3-dimethylbutyl)-L-α-aspartyl]-L-phenylalanine 1-methyl ester of the formula : 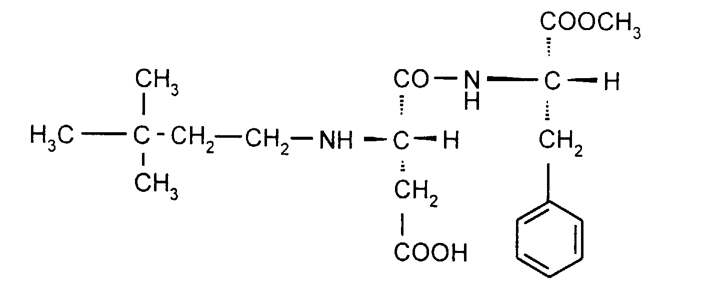 characterised in that a solution of aspartame and 3,3-dimethylbutyraldehyde, at room temperature, is treated with hydrogen at a relative pressure equal to or less than 1 bar (0.1 MPa), in the presence of a catalyst based on platinum or palladium.

2. The method according to claim 1, characterised in that the catalyst is selected from the group comprising platinum on activated carbon, palladium on activated carbon, platinum black and palladium black.

3. The method according to claim 1, characterised in that the hydrogenation is carried out in the presence of 5% platinum on activated carbon at a relative pressure of 1 bar.

4. The method according to claim 1, characterised in that the hydrogenation is carried out in the presence of 10% palladium on activated carbon at a relative pressure of 1 bar or at atmospheric pressure.

5. The method according to claim 1, characterised in that the hydrogenation is carried out in the presence of platinum black or palladium black at atmospheric pressure.

6. The method according to claim 1, characterised in that the solution of aspartame and 3,3-dimethylbutyraldehyde is an aqueous-alcoholic solution of pH 4.4-5 obtained by mixing a 0.1 M solution of acetic acid and methanol.

7. The method according to claims 1 to 6, characterised in that the concentration of aspartame in the aqueous-alcoholic solvent is between 50 and 60 g/l and the concentration of 3,3-dimethylbutyraldehyde is between 20 and 30 g/l.

8. The method according to claims 1 to 7, characterised in that the product formed is purified by precipitation and filtration after evaporation of the alcohol part of the solvent under vacuum.
